# EUROPÄISCHE PATENTSCHRIFT

(11) **EP 3 823 688 B1**
(45) Veröffentlichungstag und Bekanntmachung des Hinweises auf die Patenterteilung: **07.09.2022**
(21) Anmeldenummer: 19742588.7
(22) Anmeldetag: 19.07.2019
(51) Int. Cl.: A61M 60/135, A61M 60/857

(54) **ZULAUFLEITUNG FÜR EINE PUMPENEINHEIT EINES HERZUNTERSTÜTZUNGSSYSTEMS, HERZUNTERSTÜTZUNGSSYSTEM UND VERFAHREN ZUM HERSTELLEN EINER ZULAUFLEITUNG FÜR EINE PUMPENEINHEIT EINES HERZUNTERSTÜTZUNGSSYSTEMS**
FEED LINE FOR A PUMP UNIT OF A CARDIAC ASSISTANCE SYSTEM, CARDIAC ASSISTANCE SYSTEM AND METHOD FOR PRODUCING A FEED LINE FOR A PUMP UNIT OF A CARDIAC ASSISTANCE SYSTEM
CONDUITE D'ALIMENTATION POUR UNE UNITÉ DE POMPAGE D'UN SYSTÈME D'ASSISTANCE CARDIAQUE, SYSTÈME D'ASSISTANCE CARDIAQUE ET PROCÉDÉ DESTINÉ À FABRIQUER UNE CONDUITE D'ALIMENTATION POUR UNE UNITÉ DE POMPE D'UN SYSTÈME D'ASSISTANCE CARDIAQUE

(30) Priorität: 20.07.2018 DE 102018212153
(43) Veröffentlichungstag der Anmeldung: 26.05.2021
(73) Patentinhaber: Kardion GmbH, 70376 Stuttgart (DE)
(72) Erfinder: STOTZ, Ingo, 71254 Ditzingen (DE); EIBERGER, Fabian, 88690 Uhldingen-Mühlhofen (DE); WASSERMANN, Peter, 71263 Weil der Stadt (DE)
(74) Vertreter: Pfiz, Thomas
(86) Internationale Anmeldenummer: PCT/EP2019/069571
(87) Internationale Veröffentlichungsnummer: WO 2020/016438

(56) Entgegenhaltungen:
- US-A- 6 007 478
- US-A1- 2017 087 288

## Beschreibung

Die Erfindung geht von einer Zulaufleitung für eine Pumpeneinheit eines Herzunterstützungssystems, Herzunterstützungssystem und einem Verfahren zum Herstellen einer Zulaufleitung für eine Pumpeneinheit eines Herzunterstützungssystems nach Gattung der unabhängigen Ansprüche aus.

Eine Herzpumpe kann eine Pumpeneinheit und einen Zulaufschlauch zum Zuleiten eines Blutstroms zu der Pumpeneinheit aufweisen. Zudem kann die Herzpumpe eine Struktur zum Vermeiden oder Verringern einer Blutschädigung (einer Hämolyse) im Bereich der Pumpeneinheit aufweisen. Die US 2017/087288 A1 beschreibt eine Pumpeneinheit mit einer trompetenförmigen Vergrößerung einer Länge der Pumpeneinheit, um die Hämolyse zu reduzieren und/oder den Druckverlust zu minimieren.

Weiterer relevanter Stand der Technik ist beispielsweise in den Dokumenten US6007478 A und US2017/087288 A1 offenbart.

Ausgehend hiervon liegt der Erfindung die Aufgabe zugrunde, die im Stand der Technik bekannten Systeme und Verfahren weiter zu verbessern und dabei einen möglichst hohen Pumpwirkungsgrad bei kompakter Bauweise zu gewährleisten.

Vor diesem Hintergrund werden mit dem hier vorgestellten Ansatz eine Zulaufleitung für eine Pumpeneinheit eines Herzunterstützungssystems, ein Herzunterstützungssystem und ein Verfahren zum Herstellen einer Zulaufleitung für eine Pumpeneinheit eines Herzunterstützungssystems gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Mit diesem Ansatz wird eine Ausformung einer Zulaufleitung für eine Pumpeneinheit eines Herzunterstützungssystems vorgestellt, die durch eine Innenflächenkontur in einem Abschnitt nahe einem Einlauf der Zulaufleitung ein Strömungsverhalten eines in die Zulaufleitung eingeleiteten Fluidstroms einstellt. Mittels einer Verrundung, beispielsweise einer abschnittsweise konkaven und zusätzlich oder alternativ konvexen Ausformung der Innenflächenkontur kann vorteilhafterweise eine Strömungsablösung des Fluidstroms reduziert oder ganz vermieden werden. Mit der reduzierten oder vermiedenen Strömungsablösung sinkt auch der Druckverlust beim Leiten des Fluidstroms in der Zulaufleitung, was vorteilhaft in Bezug auf die Leistungsaufnahme einer Pumpeneinheit und damit einen Pumpenwirkungsgrad des Herzunterstützungssystems ist. Das Verringern oder Vermeiden der Strömungsablösung mittels der Innenflächenkontur ermöglicht vorteilhafterweise eine kompakte Bauweise der Zulaufleitung, was insbesondere für ein Herzunterstützungssystem zum minimalinvasiven Implantieren, beispielsweise zum transaortalen oder transfemoralen Implantieren, vorteilhaft ist.

Es wird eine Zulaufleitung für eine Pumpeneinheit eines Herzunterstützungssystems vorgestellt. Die Zulaufleitung ist ausgebildet, einen Fluidstrom zu der Pumpeneinheit des Herzunterstützungssystems zu leiten. Die Zulaufleitung weist zumindest einen Zulaufkopfabschnitt und einen Konturabschnitt auf. Der Zulaufkopfabschnitt weist zumindest eine Einleitöffnung zum Einleiten des Fluidstroms in die Zulaufleitung auf. Der Konturabschnitt weist eine Innenflächenkontur auf. Der Konturabschnitt ist benachbart zu dem Zulaufkopfabschnitt angeordnet. Ein Innendurchmesser des Konturabschnitts ist an einer ersten Position größer als der Innendurchmesser an einer zweiten Position ist. Die Innenflächenkontur weist zum Verkleinern des Innendurchmessers an der zweiten Position eine Verrundung auf.

Bei dem Herzunterstützungssystem kann es sich beispielsweise um eine Herzpumpe wie ein linksventrikuläres Unterstützungssystem, ein rechtsventrikuläres Unterstützungssystem, oder ein biventrikuläres Unterstützungssystem handeln. Das Herzunterstützungssystem kann beispielsweise zum minimalinvasiven Implantieren ausgeformt sein. Die Zulaufleitung kann als Strömungskanal zum Leiten eines Fluidstroms ausgeformt sein. Die Zulaufleitung kann auch als Schlauch ausgeformt sein. Im implantierten Zustand des Herzunterstützungssystems kann die Zulaufleitung dazu ausgeformt sein, als Fluidstrom einen Blutstrom aus einer Herzkammer zu der Pumpeneinheit des Herzunterstützungssystems zu leiten. Bei der Pumpeneinheit kann es sich beispielsweise um eine Mikroaxialpumpe handeln. Unter dem Zulaufkopfabschnitt kann ein Teilabschnitt der Zulaufleitung verstanden werden, der zwischen einer Kopfeinheit des Herzunterstützungssystems, beispielsweise einer Sensorbaugruppe, und einem benachbart der Pumpeneinheit angeordneten weiteren Teilabschnitt der Zulaufleitung angeordnet ist. Die Einleitöffnung kann beispielsweise aus der Zulaufleitung herausgeschnitten sein. Die Einleitöffnung kann als mehrteilige Fensteröffnung ausgeformt sein, beispielsweise durch drei in die Zulaufleitung eingeschnittene in Richtung der Pumpeneinheit abgerundete Fenster. Unter dem Konturabschnitt kann ein zwischen dem Zulaufkopfabschnitt und dem weiteren Teilabschnitt der Zulaufleitung angeordneter Abschnitt der Zulaufleitung verstanden werden. Bei der Innenflächenkontur kann es sich beispielsweise um eine eingeschnittene oder aufgetragenen Struktur handeln, oder um eine vordefinierte Materialausformung wie eine abschnittsweise unterschiedliche Wandstärke, oder um ein Einlegeelement, das eine bestimmte Form oder Struktur aufweist. Die erste Position kann in Bezug auf eine Strömungsrichtung des Fluidstroms der zweiten Position vorgelagert sein. Die Verrundung der Innenflächenkontur kann eine konvexe Ausbuchtung sein. Zudem kann die Innenflächenkontur an der ersten Position eine weitere Verrundung beispielsweise in Form einer konkaven Einbuchtung aufweisen.

Erfindungsgemäß ist der Innendurchmesser des Konturabschnitts an einer dritten Position größer als der Innendurchmesser an der zweiten Position. Die zweite Position befindet sich zwischen der dritten Position und der ersten Position. Die dritte Position kann somit in Bezug auf eine Strömungsrichtung des Fluidstroms der zweiten Position nachgelagert sein. Entsprechend kann die Innenflächenkontur beispielsweise an der dritten Position eine Verrundung in Form einer weiteren konkaven Einbuchtung aufweisen. Vorteilhafterweise kann dadurch das Strömungsverhalten des Fluidstroms verändert werden, was die Strömungsablösung des Fluidstroms verringert. Dies ist von Vorteil, um den Druckverlust oder die Reibung des Fluidstroms zu verringern, was den Pumpenwirkungsgrad des Herzunterstützungssystems erhöhen kann.

Die erste Position kann gemäß einer Ausführungsform im Konturabschnitt zwischen dem Zulaufkopfabschnitt und der zweiten Position angeordnet sein. Die erste Position und die zweite Position können beispielsweise Abschnitte entlang einer Längsachse des Konturabschnitts sein. Vorteilhafterweise kann durch diese Anordnung der Innendurchmesser des Konturabschnitts entlang der Längsachse des Konturabschnitts verändert werden, um durch eine Formoptimierung das Strömungsverhalten und damit die Strömungsablösung des Fluidstroms einzustellen.

Zusätzlich oder alternativ können die erste Position und die zweite Position gemäß einer Ausführungsform entlang des Umfangs des Konturabschnitts angeordnet sein. Entsprechend kann auch ein Querschnitt des Konturabschnitts zumindest eine Verrundung aufweisen, wodurch vorteilhafterweise auch das Strömungsverhalten des Fluidstroms eingestellt werden kann.

Eine Länge des Konturabschnitts kann gemäß einer Ausführungsform bis zu dem Doppelten des Innendurchmessers des Konturabschnitts entsprechen. Insbesondere kann die Länge des Konturabschnitts einem Radius der Zulaufleitung innerhalb eines Toleranzbereichs entsprechen. Die Zulaufleitung kann einen konstanten Radius aufweisen. Unter dem Toleranzbereich kann beispielsweise eine Abweichung um maximal zwanzig Prozent von dem Radius der Zulaufleitung verstanden werden. Damit ist ein lokal begrenztes Einstellen des Strömungsverhaltens möglich. Vorteilhafterweise fällt der Druckverlust weiter stromabwärts der Zulaufleitung, also in einem Teilabschnitt der Zulaufleitung zwischen dem Konturabschnitt und der Pumpeneinheit, dadurch auch geringer aus, da sich durch die Unterdrückung der Ablösung stromabwärts eine geringere Turbulenz des Fluidstroms einstellt.

Erfindungsgemäß ist ein Innenradius des Konturabschnitts an der zweiten Position maximal ein Fünftel kleiner als der Innenradius an der ersten Position. Vorteilhafterweise kann somit das Strömungsverhalten eingestellt werden, und gleichzeitig kann der zu fördernde Fluidstrom ohne Bauraumvergrößerung durch den Konturabschnitt geleitet werden.

Zudem kann die Innenflächenkontur rotationssymmetrisch ausgeformt sein. Dies ist beispielsweise im Falle einer rotationssymmetrischen Einströmung in die Zulaufleitung zur Minimierung der Strömungsablösung und damit zur Senkung des Druckverlustes vorteilhaft.

Eine Wandstärke des Konturabschnitts kann gemäß einer Ausführungsform an der ersten Position geringer als die Wandstärke an der zweiten Position sein. Alternativ oder zusätzlich kann die Innenflächenkontur als Einlegeelement des Konturabschnitts ausgeformt sein. Beide Ausführungsformen ermöglichen vorteilhafterweise eine einfache und kostengünstige Realisierung des Konturabschnitts.

Ferner kann gemäß einer Ausführungsform zumindest eine Einlaufkante der Einleitöffnung des Zulaufkopfabschnitts abgerundet sein. Vorteilhafterweise wird dadurch der Druckverlust an der Einleitöffnung reduziert.

Außerdem kann der Konturabschnitt gemäß einer Ausführungsform einen konstanten Außendurchmesser aufweisen. Dies ermöglicht vorteilhafterweise eine kompakte Bauweise, was insbesondere bei einer Verwendung der Zulaufleitung in Verbindung mit einem Herzunterstützungssystem zum minimalinvasiven Implantieren vorteilhaft ist.

Es wird zudem ein Herzunterstützungssystem mit einer Ausführungsform der vorstehend genannten Zulaufleitung vorgestellt.

Zudem wird ein Verfahren zum Herstellen einer Zulaufleitung für ein Herzunterstützungssystem vorgestellt. Die Zulaufleitung ist ausgebildet, einen Fluidstrom zu einer Pumpeneinheit des Herzunterstützungssystems zu leiten. Das Verfahren umfasst einen Schritt des Ausformens. Im Schritt des Ausformens wird ein Zulaufkopfabschnitt mit zumindest einer Einleitöffnung zum Einleiten des Fluidstroms in die Zulaufleitung ausgeformt. Ferner wird im Schritt des Ausformens ein Konturabschnitt mit einer Innenflächenkontur ausgeformt, der Konturabschnitt benachbart zu dem Zulaufkopfabschnitt angeordnet ist, wobei ein Innendurchmesser des Konturabschnitts an einer ersten Position größer als der Innendurchmesser an einer zweiten Position ist und wobei die Innenflächenkontur zum Verkleinern des Innendurchmessers an der zweiten Position eine Verrundung aufweist.

Die Zulaufleitung kann beispielsweise aus einem Rohr geschnitten werden, oder die Zulaufleitung kann als Schlauch ausgeformt sein und ein Einlegeelement zum Ausformen des Konturabschnitts und der Innenflächenkontur aufweisen. Durch Ausführen des Verfahrens ist eine Ausführungsform der vorstehend genannten Gleitlagervorrichtung vorteilhaft herstellbar.

Ausführungsbeispiele des hier vorgestellten Ansatzes sind in den Zeichnungen dargestellt und in der nachfolgenden Beschreibung näher erläutert. Es zeigt:
- Fig. 1: eine schematische Darstellung eines Herzunterstützungssystems mit einer Zulaufleitung;
- Fig. 2: eine schematische Darstellung eines Teils eines Konturabschnitts einer Zulaufleitung gemäß einem Ausführungsbeispiel;
- Fig. 3: eine schematische Darstellung eines Teils einer Zulaufleitung gemäß einem Ausführungsbeispiel;
- Fig. 4: eine schematische Darstellung eines Teils einer Zulaufleitung gemäß einem Ausführungsbeispiel;
- Fig. 5: eine Visualisierung der Auswirkung der vorgenommen Verrundung im Vergleich zu einem Schlauch mit einem konstanten Innendurchmesser;
- Fig. 6: eine Visualisierung der Auswirkung der vorgenommen Verrundung im Vergleich zu einem Schlauch mit einem konstanten Innendurchmesser;
- Fig. 7: eine schematische Darstellung eines Teils eines Konturabschnitts einer Zulaufleitung gemäß einem Ausführungsbeispiel;
- Fig. 8: eine schematische Darstellung eines Teils einer Innenflächenkontur eines Konturabschnitts einer Zulaufleitung gemäß einem Ausführungsbeispiel; und
- Fig. 9: ein Ablaufdiagramm eines Verfahrens zum Herstellen einer Zulaufleitung für ein Herzunterstützungssystem gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

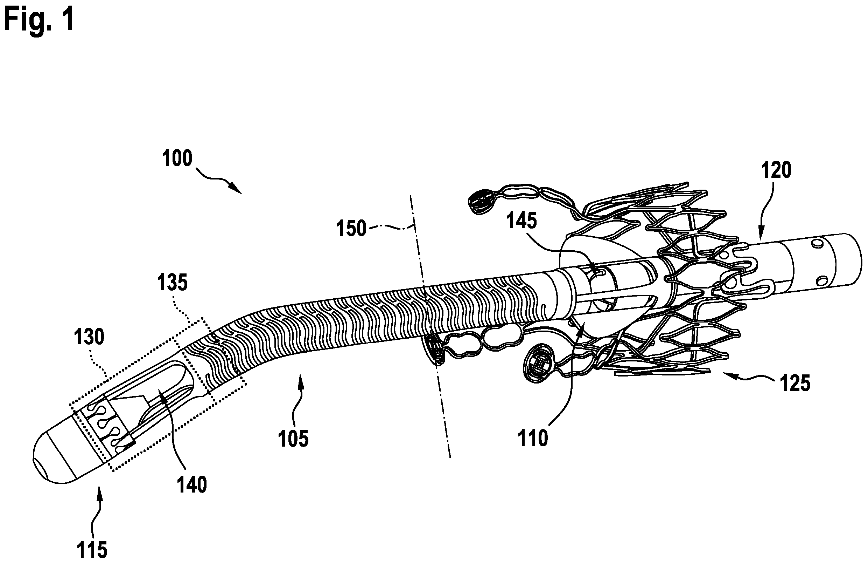

Fig. 1 zeigt eine schematische Darstellung eines Herzunterstützungssystems 100 mit einer Zulaufleitung 105 gemäß einem Ausführungsbeispiel. Es ist eine perspektivische Ansicht des Herzunterstützungssystems 100 gezeigt, das beispielhaft als linksventrikuläres Herzunterstützungssystem, als "left ventricular assist device" (LVAD) für eine Aortenklappenposition ausgeführt ist. Die hier gezeigte längliche axiale Bauart des Herzunterstützungssystems 100 mit im Wesentlichen konstanten Außendurchmesser ermöglicht eine transfemorale oder transaortale Implantation des Herzunterstützungssystems 100 zur einfachen Platzierung mittels eines Katheters in einem Blutgefäß, etwa der Aorta.

Die Zulaufleitung 105 weist entsprechend der Ausformung für die Aortenklappenposition beispielhaft eine Neigung bzw. Krümmung der Längsachse und damit eine leicht gebogene Form auf. Das Herzunterstützungssystem umfasst außer der Zulaufleitung 105 eine Pumpeneinheit 110. Beispielhaft sind zudem eine Kopfeinheit 115 sowie ein Gehäuseabschnitt 120 und ein Verankerungsrahmen 125 gezeigt. Die Zulaufleitung 105 ist zwischen der Kopfeinheit 115 und der Pumpeneinheit 110 angeordnet. Die Pumpeneinheit 110 ist an einem der Zulaufleitung 105 abgewandtem Ende mit dem Gehäuseabschnitt 120 verbunden an dem der Verankerungsrahmen 125 befestigt ist.

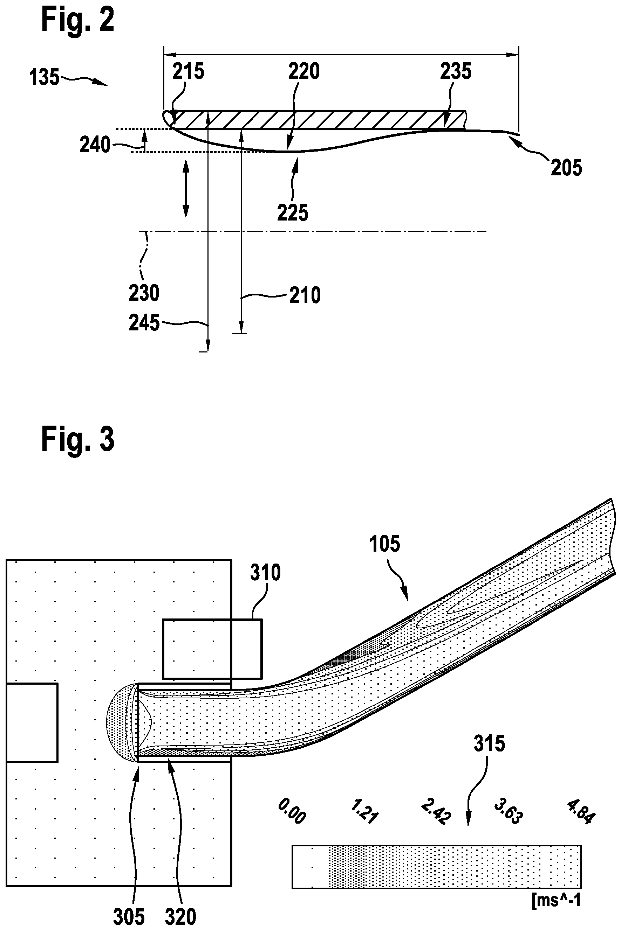

Die Zulaufleitung 105 ist gemäß einem Ausführungsbeispiel ausgebildet, einen Fluidstrom zu der Pumpeneinheit 110 des Herzunterstützungssystems 100 zu leiten. Die Zulaufleitung umfasst einen Zulaufkopfabschnitt 130 und einen Konturabschnitt 135. Der Zulaufkopfabschnitt 130 weist zumindest eine Einleitöffnung 140 zum Einleiten des Fluidstroms in die Zulaufleitung 105 auf. Der Konturabschnitt 135 weist eine Innenflächenkontur auf. Der Konturabschnitt 135 ist benachbart zu dem Zulaufkopfabschnitt 130 angeordnet. In Durchflussrichtung gesehen ist der Innendurchmesser des Konturabschnitts 135 an einer ersten Position größer als der Innendurchmesser an einer zweiten Position. Die Innenflächenkontur weist zum Verkleinern des Innendurchmessers an der zweiten Position eine Verrundung auf. Ausführungsbeispiele von Ausformungen des Konturabschnitts 135 sind anhand der nachfolgenden Figuren 2 bis 9 beschrieben.

In dem hier gezeigten Ausführungsbeispiel sind der Zulaufkopfabschnitt 130 und der Konturabschnitt 135 beispielhaft markiert. Insbesondere der Konturabschnitt 135 umfasst optional einen geringeren oder größeren Anteil der Zulaufleitung 105 als hier gezeigt. Im implantierten Zustand des Herzunterstützungssystems 100 sind der Zulaufkopfabschnitt 130 und der Konturabschnitt 135 in der linken Herzkammer angeordnet. Ein weiterer Abschnitt der Zulaufleitung 105 ist durch die Aortenklappe geführt, und ein Abschnitt des Herzunterstützungssystems 100 mit der Pumpeneinheit 110 ist in einem Abschnitt der Aorta angeordnet. Ein Pumpenauslauf 145 im Bereich der Pumpeneinheit 110 leitet den durch die Zulaufleitung 105 geförderten Fluidstrom in die Aorta. Die Markierung 150 zeigt beispielhaft eine Lage einer Herzklappe, beispielsweise der Aortenklappe, durch die die Zulaufleitung 105 durchgeführt ist, um das Herzunterstützungssystem 100 zu positionieren.

Zumindest eine Einlaufkante der Einleitöffnung 140 des Zulaufkopfabschnitts 130 ist gemäß dem hier gezeigten Ausführungsbeispiel abgerundet. Die Einleitöffnung 140 ist hier beispielhaft als in den Zulaufkopfabschnitt 103 eingeschnittener fensterförmiger Zulauf ausgeführt.

Gemäß dem hier gezeigten Ausführungsbeispiel entspricht eine Länge des Konturabschnitts 135 einem Radius der Zulaufleitung 105 innerhalb eines Toleranzbereichs. Unter dem Toleranzbereich ist eine Abweichung um maximal zwanzig Prozent von dem Radius der Zulaufleitung zu verstehen.

Ein in Bezug auf den Bauraum limitiertes Herzunterstützungssystem, wie das hier beispielhaft gezeigte Herzunterstützungssystem 100, das minimalinvasiv implantiert werden kann, weist eine vergleichsweise geringe Leistungsaufnahme bei einem bestimmten Pumpenwirkungsgrad auf. Der Wirkungsgrad wird durch die Reibung in der Pumpe der Pumpeneinheit 110 begrenzt. Der Druckverlust oder die Reibung in der Zulaufleitung 105 bei dem Leiten des Fluidstroms von der Einleitungsöffnung 140 des Zulaufkopfabschnittes 130 in der Herzkammer zu der Pumpeneinheit 110 ist durch die Ausformung der Zulaufleitung 105 einstellbar. Dazu sind die Einlaufkanten der Einlassöffnung 140 abgerundet ausgeführt um, um den Druckverlust zu reduzieren. Die Strömungsablösung ist ausschließlich dadurch nicht zu verhindern. Durch eine gemäß dem hier vorgestellten Ansatz ausgeformte Einlaufinnenflächenkontur in Form des Konturabschnitts 135 wird die Strömungsablösung unterdrückt und damit der Druckverlust gesenkt. Ausführungsbeispiele einer entsprechend ausgeformten Einlaufinnenflächenkontur sind im Folgenden erläutert.

Fig. 2 zeigt eine schematische Darstellung eines Teils eines Konturabschnitts 135 einer Zulaufleitung gemäß einem Ausführungsbeispiel. Gezeigt sind beispielhafte Abmessungsrelationen des Konturabschnitts 135 und der Innenflächenkontur 205. Es ist ein axialer Schnitt einer Hälfte des Konturabschnitts 135 gezeigt. Der Innendurchmesser 210 des Konturabschnitts 135 ist an einer ersten Position 215 größer als der Innendurchmesser 210 an einer zweiten Position 220. Die Innenflächenkontur 205 weist zum Verkleinern des Innendurchmessers 210 an der zweiten Position 220 eine Verrundung 225 in Form eines axial bogenförmigen Innenwandverlaufs auf. Die erste Position 215 markiert gemäß dem hier gezeigten Ausführungsbeispiel eine Stelle des Konturabschnitts 135 entlang einer Längsachse des Konturabschnitts 135, und die zweite Position 215 markiert eine weitere Stelle des Konturabschnitts 135 entlang der Längsachse. In dem hier gezeigten Ausführungsbeispiel entspricht die Längsachse einer Rotationsachse 230 des Konturabschnitts 135.

Gemäß dem hier gezeigten Ausführungsbeispiel ist die erste Position 215 im Konturabschnitt 135 zwischen dem Zulaufkopfabschnitt und der zweiten Position 220 angeordnet. In Bezug auf eine Strömungsrichtung des durch den Zulaufkopfabschnitt eingeleiteten Fluidstroms, der in Richtung der Pumpeinheit durch die Zulaufleitung und damit durch den Konturabschnitt 135 geleitet wird, ist die erste Position 215 der zweiten Position 220 vorgelagert angeordnet. Zudem ist der Innendurchmesser des Konturabschnitts 135 gemäß dem hier gezeigten Ausführungsbeispiel an einer dritten Position 235 größer als der Innendurchmesser an der zweiten Position 220.

Ein Innenradius des Konturabschnitts 135 ist erfindungsgemäß an der zweiten Position 220 maximal ein Fünftel kleiner als der Innenradius an der ersten Position 215. In der vorliegenden Fig. 2 ist dies durch die Markierung 240 gezeigt, die ein Fünftel des Innenradius markiert. Entsprechend ist die Verrundung 225 der Innenflächenkontur 205 maximal als konvexe Ausbuchtung im Bereich von zu einem Fünftel des Innenradius ausgeführt, was die Markierung 240 zusätzlich veranschaulicht.

Die Innenflächenkontur 205 ist gemäß einem Ausführungsbeispiel rotationssymmetrisch ausgeführt. Ein dem hier gezeigten Teil der Innenflächenkontur 205 in Bezug auf die Rotationsachse 230 gegenüberliegender Teil des Konturabschnitts 135 weist entsprechend eine Rotation symmetrisch ausgeführte Innenflächenkontur 205 auf.

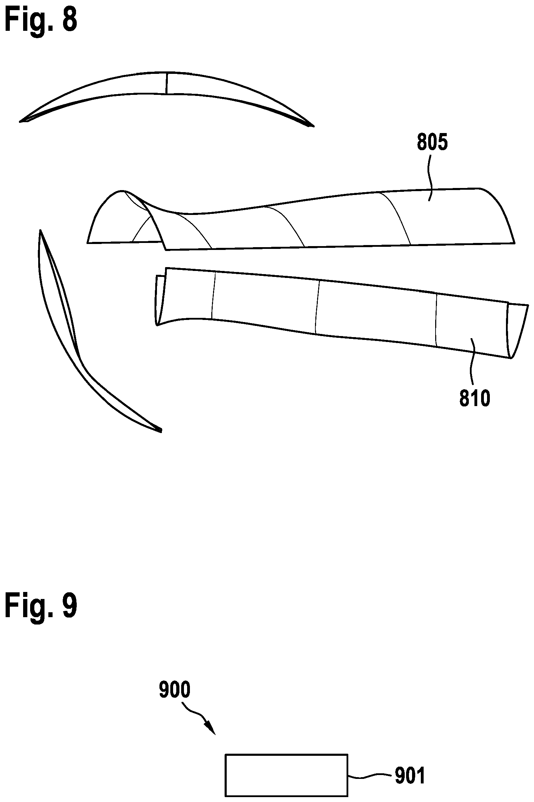

Mittels der hier gezeigten Ausformungen des Konturabschnitts 135 und der Innenflächenkontur 205 ist es möglich, Strömungsablösungen des Fluidstroms in der Zulaufleitung zu verringern oder zu unterdrücken, die sich andernfalls stromabwärts der Einlaufkanten bilden. Dabei bleibt ein Außendurchmesser 245 des Konturabschnitts 135 konstant, und eine Vergrößerung des Bauraums der Zulaufleitung unterbleibt vorteilhafterweise. Mittels einem Ausführungsbeispiel des hier gezeigten Konturabschnitts und 135 mit der Innenflächenkontur 205 wird der Druckverlust des zu fördernden Fluidstroms gesenkt. Dabei wird die Einlaufströmungen und damit das Strömungsverhalten des Fluidstroms nur lokal durch den Konturabschnitt 135 gelenkt. Der Konturabschnitt 135 weist optional eine Länge auf, die in diesem Ausführungsbeispiel maximal einem Doppelten Innendurchmessers der Zulaufleitung entspricht. Der Druckverlust des Fluidstroms fällt durch die Ausformung des Konturabschnitts 135 weiter stromabwärts geringer aus als in einer Zulaufleitung mit konstantem Innendurchmesser ohne Innenflächenkontur, da sich durch eine Unterdrückung bzw. Verringerung der Ablösung eine geringere Turbulenz stromabwärts einstellt. Dabei ist die Innenflächenkontur 205 so ausgeformt, dass die Strömungsablösung auf einer Länge von bis zu vier Mal dem Radius der Zulaufleitung weitgehend unterdrückt wird. Der lokale Außendurchmesser 245 der Zulaufleitung ist dabei durch eine vorgeschriebene Wandstärke begrenzt. Angrenzend an die Einleitungsöffnung des Zulaufkopfabschnitts ist die Einlaufkante zum Verringern der Strömungsablösung konvex verrundet. Eine Formoptimierung der Innenflächenkontur 205 wie die hier gezeigte Ausformung ist optional rotationssymmetrisch oder alternativ unabhängig vom Drehwinkel, wie anhand der Fig. 8 gezeigt. Gemäß dem hier gezeigten Ausführungsbeispiel weist ein Konturprofil der Innenflächenkontur 205 unabhängig von der beschriebenen Einlaufkantenverrundung bei einer konstanten Wandstärke zwei konkave und eine konvexe Formoptimierung auf, wie hier anhand der ersten Position 215, der zweiten Position 220, der dritten Position 235 und der Verrundung 225 gezeigt. Optional wird dazu die Wandinnenkontur so ausgeformt, dass lokal ein Wandinnenradius von bis zu vier Fünftel bezogen auf den Wandinnenradius bei einer konstanten Wandstärke des Konturabschnitts 135 erreicht wird.

Fig. 3 zeigt eine schematische Darstellung eines Teils einer Zulaufleitung 105 gemäß einem Ausführungsbeispiel. Es ist eine Visualisierung einer Strömungsablösung 305 an einer Einlaufkante im Einlaufbereich 310 anhand einer Strömungsgeschwindigkeit eines durch die Zulaufleitung 105 geförderten Fluidstroms gezeigt. Der hier gezeigte Einlaufbereich 310 entspricht einem Ausschnitt eines an die Einleitöffnung angrenzenden Abschnitts der Zulaufleitung. Die Skala 315 zeigt die Strömungsgeschwindigkeit des Fluidstroms. Die Strömungsablösung 305 ist auch anhand der durch die Markierung 320 gezeigten Linie, an der die Strömungsgeschwindigkeit gleich Null ist, gezeigt. Der durch die Markierung 320 gezeigte Abschnitt im Einlaufbereich 310 kann entsprechend auch als "Nullgeschwindigkeitslinie" bezeichnet werden. Die hier gezeigte Strömungsablösung 305 des Fluidstroms ist mittels einem Ausführungsbeispiel der Innenflächenkontur und des Konturabschnitts, wie sie anhand vorstehender und nachfolgender Figuren beschrieben sind, reduzierbar.

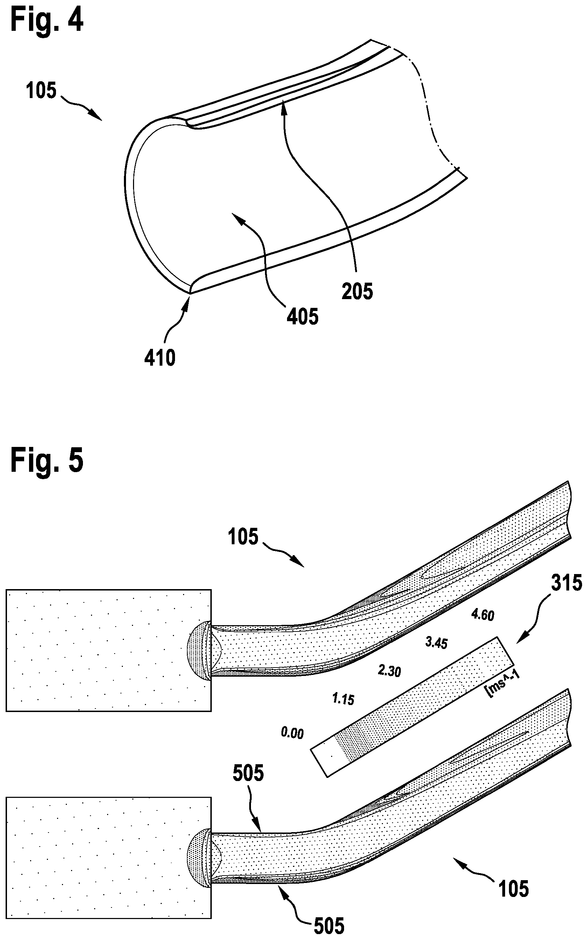

Fig. 4 zeigt eine schematische Darstellung eines Teils einer Zulaufleitung 105 gemäß einem Ausführungsbeispiel. Es ist ein Querschnitt der Zulaufleitung 105 gezeigt, die der anhand der vorstehenden Figuren beschriebenen Zulaufleitung ähnelt oder entspricht. Fig. 4 zeigt eine Formoptimierung im Zulaufbereich der Zulaufleitung 105. Der hier gezeigte Abschnitt der Zulaufleitung 105 zeigt den an die Einleitöffnung des Zulaufkopfabschnitts angrenzenden Einlaufbereich 405 des Konturabschnitts. Entsprechend ist ein Bereich 410 angrenzend an die Einlaufkante abgerundet. Zudem ist die Innenflächenkontur 205 gezeigt. Die Abrundung im Bereich 410 und die Ausformung der Innenflächenkontur 205 können in Bezug auf die Verringerung der Strömungsablösung auch als lokale Topologieanpassung im Einlaufbereich bezeichnet werden.

Fig. 5 zeigt eine schematische Darstellung eines Teils einer Zulaufleitung 105 gemäß einem Ausführungsbeispiel. Es sind zwei Situationen des Strömungsverhaltens des Fluidstroms anhand der durch die Strömungsgeschwindigkeit erkennbare Strömungsablösung in der Zulaufleitung 105 gezeigt. Die Skala 315 zeigt die Strömungsgeschwindigkeit des Fluidstroms. Die oben gezeigte Zulaufleitung 105 zeigt beispielhaft die Strömungsgeschwindigkeit des Fluidstroms in einer Zulaufleitung 105 mit einer Strömungsablösung im Einlaufbereich, die anhand der "Nullgeschwindigkeitslinien" wie anhand von Fig. 3 beschrieben erkennbar ist. Die unten gezeigte Situation der Zulaufleitung 105 zeigt beispielhaft die Strömungsgeschwindigkeit des Fluidstroms, bei dem die Strömungsablösung im Einlaufbereich verhindert wird, wie durch die Markierung 505 gezeigt. Die unten gezeigte Situation ähnelt oder entspricht der anhand von Fig. 2 beschriebenen Ausformung des Konturabschnitts und der Innenflächenkontur der Zulaufleitung 105.

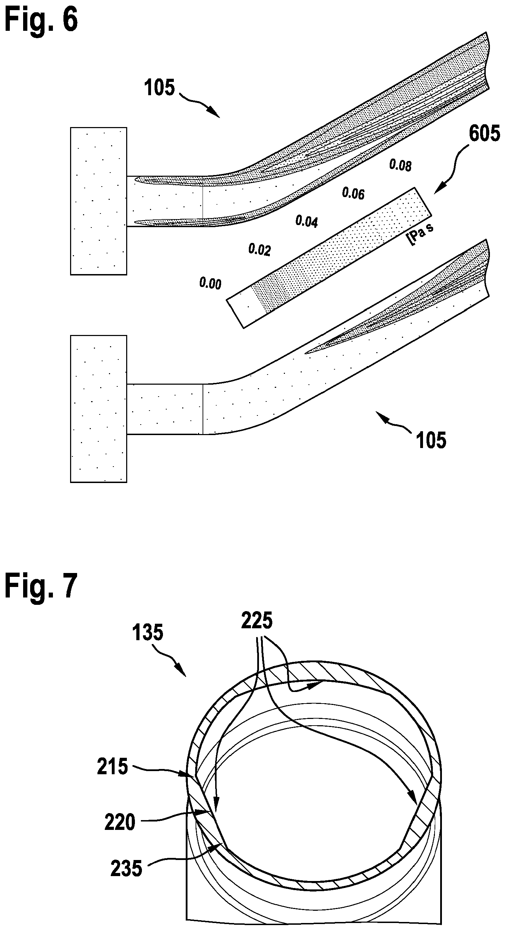

Fig. 6 zeigt eine schematische Darstellung eines Teils einer Zulaufleitung 105 gemäß einem Ausführungsbeispiel. Es sind zwei Situationen des Strömungsverhaltens des Fluidstroms anhand der scheinbaren Viskosität der turbulenten Strömung, der "Wirbelviskosität", die durch die Skala 605 visualisiert wird, gezeigt. Anhand der Darstellung der Viskosität der turbulenten Strömung ist auch die Reibung des Fluidstroms im Einlaufbereich der Zulaufleitung 105 gezeigt. Die oben gezeigte Zulaufleitung 105 zeigt beispielhaft die "Wirbelviskosität" des Fluidstroms in einer Zulaufleitung 105 mit einer Strömungsablösung im Einlaufbereich. Die unten gezeigte Situation der Zulaufleitung 105 zeigt beispielhaft die Reduktion der Reibung auch im Bereich stromabwärts des Einlaufbereichs der Zulaufleitung 105. Die unten gezeigte Situation ähnelt oder entspricht der anhand von Fig. 2 beschriebenen Ausformung des Konturabschnitts und der Innenflächenkontur der Zulaufleitung 105.

Fig. 7 zeigt eine schematische Darstellung eines Teils eines Konturabschnitts 135 einer Zulaufleitung gemäß einem Ausführungsbeispiel. Es ist ein Querschnitt des Konturabschnitts 135 gezeigt. Der hier gezeigte Konturabschnitt 135 ähnelt oder entspricht dem Konturabschnitt wie er anhand von Fig. 2 beschrieben ist. Fig. 7 zeigt hierbei ebenfalls ein Beispiel, bei dem die Verrundung nicht rotationssymmetrisch ist.

Fig. 8 zeigt eine schematische Darstellung eines Teils einer Innenflächenkontur 205 eines Konturabschnitts einer Zulaufleitung gemäß einem Ausführungsbeispiel. Gemäß dem hier gezeigten Ausführungsbeispiel ist die Innenflächenkontur 205 als Einlegeelement des Konturabschnitts ausgeformt. Beispielhaft ist die Innenflächenkontur 205 hier als zweiteiliges Einlegeelement mit zwei nicht-rotationssymmetrischen Innenflächenkontur-Teilen 805, 810 ausgeführt, die zusammen die Innenflächenkontur 205 des Konturabschnitts ausformen. Das zweiteilige Einlegeelement kann als Beispiel des Falles einer Einlassöffnung 140 mit zwei offenen Fenstern und einem geschlossen Fenster dienen. In der hier gezeigten Darstellung sind verschiedene Ansichten der Innenflächenkontur 205 als Einlegeelement gezeigt: Die obere Ansicht zeigt einen Querschnitt durch das Innenflächenkontur-Teil 805. In der Mitte sind die beiden Innenflächenkontur-Teile 805 und 810 in einer Seitenansicht gezeigt und in der unteren Darstellung ist ein Querschnitt durch das Innenflächenkontur-Teil 810 gezeigt. Die hier gezeigten Innenflächenkontur-Teile 805 und 810 formen einzeln oder zusammengefügt die Innenflächenkontur 205 des Konturabschnitts der Zulaufleitung aus. Sie können entsprechend als nicht-rotationssymmetrische Optimierung zur Zulaufleitung zum Verringern der Strömungsablösung bezeichnet werden.

Fig. 9 zeigt ein Ablaufdiagramm eines Verfahrens 900 zum Herstellen einer Zulaufleitung für eine Pumpeneinheit eines Herzunterstützungssystems gemäß einem Ausführungsbeispiel. Die Zulaufleitung ist ausgebildet, einen Fluidstrom zu der Pumpeneinheit des Herzunterstützungssystems zu leiten. Das Verfahren 900 weist einen Schritt 901 des Ausformens auf. Im Schritt 905 des Ausformens wird ein Zulaufkopfabschnitt mit zumindest einer Einleitöffnung zum Einleiten des Fluidstroms in die Zulaufleitung ausgeformt. Zudem wird ein Konturabschnitt mit einer Innenflächenkontur ausgeformt, wobei der Konturabschnitt benachbart zu dem Zulaufkopfabschnitt angeordnet ist. Ein Innendurchmesser des Konturabschnitts ist an einer ersten Position größer als der Innendurchmesser an einer zweiten Position. Die Innenflächenkontur weist zum Verkleinern des Innendurchmessers an der zweiten Position eine Verrundung auf.

Im Schritt 901 des Ausformens werden der Zulaufkopfabschnitt und der Konturabschnitt aus einem Rohr geschnitten. Zusätzlich oder alternativ wird die Innenflächenkontur des Konturabschnitts als Einlegeelement der Zulaufleitung ausgeformt.

## Patentansprüche

1. Zulaufleitung (105) für eine Pumpeneinheit (110) eines Herzunterstützungssystems (100), wobei die Zulaufleitung (105) ausgebildet ist, einen Fluidstrom zu der Pumpeneinheit (110) des Herzunterstützungssystems (100) zu leiten, wobei die Zulaufleitung (105) folgende Merkmale aufweist:
einen Zulaufkopfabschnitt (130) mit zumindest einer Einleitöffnung (140) zum Einleiten des Fluidstroms in die Zulaufleitung (105); und
einen Konturabschnitt (135) mit einer Innenflächenkontur (205), wobei der Konturabschnitt (135) benachbart zu dem Zulaufkopfabschnitt (130) angeordnet ist, wobei ein Innendurchmesser (210) des Konturabschnitts (135) an einer ersten Position (215) größer als der Innendurchmesser (210) an einer zweiten Position (220) ist, wobei der Innendurchmesser (210) des Konturabschnitts an der ersten Position (215) maximal und an der zweiten Position (220) minimal ist und die Innenflächenkontur (205) zum Verkleinern des Innendurchmessers (210) an der zweiten Position (220) eine Verrundung (225) aufweist,
**dadurch gekennzeichnet, dass** ein Innenradius des Konturabschnitts (135) an der zweiten Position (220) maximal ein Fünftel kleiner als der Innenradius an der ersten Position (215) ist, wobei der Innendurchmesser (210) des Konturabschnitts (135) an einer dritten Position (235) größer als der Innendurchmesser (210) an der zweiten Position (220) ist, wobei sich die zweite Position (220) zwischen der dritten Position (235) und der ersten Position (215) befindet.

2. Zulaufleitung (105) gemäß Anspruch 1, wobei die erste Position (215) im Konturabschnitt (135) zwischen dem Zulaufkopfabschnitt (130) und der zweiten Position (220) angeordnet ist.

3. Zulaufleitung (105) gemäß einem der vorangegangenen Ansprüche, wobei eine Länge des Konturabschnitts (135) bis zu dem Doppelten des Innendurchmessers (210) des Konturabschnitts (135) entspricht, insbesondere wobei die Länge des Konturabschnitts (135) einem Radius der Zulaufleitung (105) innerhalb eines Toleranzbereichs entspricht.

4. Zulaufleitung (105) gemäß einem der vorangegangenen Ansprüche, wobei die Innenflächenkontur (205) rotationssymmetrisch ausgeformt ist.

5. Zulaufleitung (105) gemäß einem der vorangegangenen Ansprüche, wobei eine Wandstärke des Konturabschnitts (135) an der ersten Position (215) geringer ist als die Wandstärke an der zweiten Position (220) und/oder wobei die Innenflächenkontur (205) als Einlegeelement (805, 810) des Konturabschnitts (135) ausgeformt ist.

6. Zulaufleitung (105) gemäß einem der vorangegangenen Ansprüche, wobei zumindest eine Einlaufkante der Einleitöffnung (140) des Zulaufkopfabschnitts (130) abgerundet ist.

7. Zulaufleitung (105) gemäß einem der vorangegangenen Ansprüche, wobei der Konturabschnitt (135) einen konstanten Außendurchmesser (245) aufweist.

8. Herzunterstützungssystem (100) mit einer Zulaufleitung (105) gemäß einem der vorangegangenen Ansprüche 1 bis 7.

9. Verfahren (900) zum Herstellen einer Zulaufleitung (105) für Pumpeneinheit (110) eines Herzunterstützungssystem (100) gemäß einem der vorangegangenen Ansprüche, wobei die Zulaufleitung (105) ausgebildet ist, einen Fluidstrom zu der Pumpeneinheit (110) des Herzunterstützungssystems (100) zu leiten, wobei das Verfahren (900) den folgenden Schritt aufweist:
Ausformen (901) eines Zulaufkopfabschnitts (130) mit zumindest einer Einleitöffnung (140) zum Einleiten des Fluidstroms in die Zulaufleitung (105) und Ausformen eines Konturabschnitts (135) mit einer Innenflächenkontur (205), wobei der Konturabschnitt (135) benachbart zu dem Zulaufkopfabschnitt (130) angeordnet ist, wobei ein Innendurchmesser (210) des Konturabschnitts (135) an einer ersten Position (215) größer als der Innendurchmesser (210) an einer zweiten Position (220) ist und wobei die Innenflächenkontur (205) zum Verkleinern des Innendurchmessers (210) an der zweiten Position (220) eine Verrundung (225) aufweist, **dadurch gekennzeichnet, dass** ein Innenradius des Konturabschnitts (135) an der zweiten Position (220) maximal ein Fünftel kleiner als der Innenradius an der ersten Position (215) ausgeführt wird, wobei der Innendurchmesser (210) des Konturabschnitts (135) an einer dritten Position (235) größer als der Innendurchmesser (210) an der zweiten Position (220) ist und sich die zweite Position (220) zwischen der dritten Position (235) und der ersten Position (215) befindet.

## Claims

1. Feed line (105) for a pump unit (110) of a cardiac assistance system (100), the feed line (105) being designed to conduct a fluid flow to the pump unit (110) of the cardiac assistance system (100), the feed line (105) comprising the following features:
a feed head portion (130) having at least one inlet opening (140) for introducing the fluid flow into the feed line (105); and
a contour portion (135) having an inner surface contour (205), the contour portion (135) being arranged adjacent to the feed head portion (130), an inner diameter (210) of the contour portion (135) at a first position (215) being greater than the inner diameter (210) at a second position (220), the inner diameter (210) of the contour portion being at the maximum at the first position (215) and the inner diameter being at the minimum at the second position (220) and the inner surface contour (205) having a rounded part (225) for reducing the inner diameter (210) at the second position (220),
**characterized in that** an inner radius of the contour portion (135) at the second position (220) is at most one fifth smaller than the inner radius at the first position (215), the inner diameter (210) of the contour portion (135) at a third position (235) being greater than the inner diameter (210) at the second position (220), the second position (220) being between the third position (235) and the first position (215).

2. Feed line (105) according to claim 1, wherein the first position (215) in the contour portion (135) is arranged between the feed head portion (130) and the second position (220).

3. Feed line (105) according to either of the preceding claims, wherein a length of the contour portion (135) corresponds to up to twice the inner diameter (210) of the contour portion (135), in particular wherein the length of the contour portion (135) corresponds to a radius of the feed line (105) within a tolerance range.

4. Feed line (105) according to any of the preceding claims, wherein the inner surface contour (205) is formed in a rotationally symmetrical manner.

5. Feed line (105) according to any of the preceding claims, wherein a wall thickness of the contour portion (135) at the first position (215) is less than the wall thickness at the second position (220) and/or wherein the inner surface contour (205) is formed as an insert element (805, 810) of the contour portion (135).

6. Feed line (105) according to any of the preceding claims, wherein at least one inlet edge of the inlet opening (140) of the feed head portion (130) is rounded.

7. Feed line (105) according to any of the preceding claims, wherein the contour portion (135) has a constant outer diameter (245).

8. Cardiac assistance system (100) comprising a feed line (105) according to any of the preceding claims 1 to 7.

9. Method (900) for producing a feed line (105) for a pump unit (110) of a cardiac assistance system (100) according to any of the preceding claims, the feed line (105) being designed to conduct a fluid flow to the pump unit (110) of the cardiac assistance system (100), the method (900) comprising the step of:
forming (901) a feed head portion (130) having at least one inlet opening (140) for introducing the fluid flow into the feed line (105) and forming a contour portion (135) having an inner surface contour (205), the contour portion (135) being arranged adjacent to the feed head portion (130), an inner diameter (210) of the contour portion (135) at a first position (215) being greater than the inner diameter (210) at a second position (220) and the inner surface contour (205) having a rounded part (225) for reducing the inner diameter (210) at the second position (220), **characterized in that** an inner radius of the contour portion (135) at the second position (220) is at most one fifth smaller than the inner radius at the first position (215), the inner diameter (210) of the contour portion (135) at a third position (235) being greater than the inner diameter (210) at the second position (220) and the second position (220) being between the third position (235) and the first position (215).

## Revendications

1. Conduite d'alimentation (105) pour une unité de pompage (110) d'un système d'assistance cardiaque (100), dans laquelle la conduite d'alimentation (105) est conçue pour conduire un écoulement de fluide vers l'unité de pompage (110) du système d'assistance cardiaque (100), dans laquelle la conduite d'alimentation (105) comporte les caractéristiques suivantes :
une section de tête d'alimentation (130) comportant au moins une ouverture d'entrée (140) et permettant d'introduire l'écoulement de fluide dans la conduite d'alimentation (105) ; et
une section de contour (135) comportant un contour de surface interne (205), dans laquelle la section de contour (135) est disposée adjacente à la section de tête d'alimentation (130), dans laquelle un diamètre interne (210) de la section de contour (135) à une première position (215) est supérieur au diamètre interne (210) à une deuxième position (220), dans laquelle le diamètre interne (210) de la section de contour est maximal à la première position (215) et minimal à la deuxième position (220) et le contour de surface interne (205) comporte un arrondi (225) permettant de réduire le diamètre interne (210) à la deuxième position (220),
**caractérisée en ce qu'**un rayon interne de la section de contour (135) à la deuxième position (220) est inférieur d'au plus un cinquième au rayon interne à la première position (215), dans laquelle le diamètre interne (210) de la section de contour (135) à une troisième position (235) est supérieur au diamètre interne (210) à la deuxième position (220), dans laquelle la deuxième position (220) se trouve entre la troisième position (235) et la première position (215).

2. Conduite d'alimentation (105) selon la revendication 1, dans laquelle la première position (215) est disposée dans la section de contour (135) entre la section de tête d'alimentation (130) et la deuxième position (220).

3. Conduite d'alimentation (105) selon l'une quelconque des revendications précédentes, dans laquelle une longueur de la section de contour (135) correspond jusqu'à deux fois le diamètre interne (210) de la section de contour (135), en particulier dans laquelle la longueur de la section de contour (135) correspond à un rayon de la conduite d'alimentation (105) dans une plage de tolérance.

4. Conduite d'alimentation (105) selon l'une quelconque des revendications précédentes, dans laquelle le contour de surface interne (205) est formé de manière symétrique en rotation.

5. Conduite d'alimentation (105) selon l'une quelconque des revendications précédentes, dans laquelle une épaisseur de paroi de la section de contour (135) à la première position (215) est inférieure à l'épaisseur de paroi à la deuxième position (220) et/ou dans laquelle le contour de surface interne (205) est formé en tant qu'élément d'insertion (805, 810) de la section de contour (135).

6. Conduite d'alimentation (105) selon l'une quelconque des revendications précédentes, dans laquelle au moins un bord d'admission de l'ouverture d'entrée (140) de la section de tête d'alimentation (130) est arrondi.

7. Conduite d'alimentation (105) selon l'une quelconque des revendications précédentes, dans laquelle la section de contour (135) présente un diamètre externe (245) constant.

8. Système d'assistance cardiaque (100) comportant une conduite d'alimentation (105) selon l'une quelconque des revendications précédentes 1 à 7.

9. Procédé (900) de fabrication d'une conduite d'alimentation (105) pour une unité de pompage (110) d'un système d'assistance cardiaque (100) selon l'une quelconque des revendications précédentes, dans lequel la conduite d'alimentation (105) est conçue pour conduire un écoulement de fluide vers l'unité de pompage (110) du système d'assistance cardiaque (100), dans lequel le procédé (900) comporte l'étape suivante :
la formation (901) d'une section de tête d'alimentation (130) comportant au moins une ouverture d'entrée (140) et permettant d'introduire l'écoulement de fluide dans la conduite d'alimentation (105) et la formation d'une section de contour (135) comportant un contour de surface interne (205), dans lequel la section de contour (135) est disposée adjacente à la section de tête d'alimentation (130), dans lequel un diamètre interne (210) de la section de contour (135) à une première position (215) est supérieur au diamètre interne (210) à une deuxième position (220) et dans lequel le contour de surface interne (205) comporte un arrondi (225) permettant de réduire le diamètre interne (210) à la deuxième position (220), **caractérisé en ce qu'**un rayon interne de la section de contour (135) à la deuxième position (220) est inférieur d'au plus un cinquième au rayon interne à la première position (215), dans lequel le diamètre interne (210) de la section de contour (135) à une troisième position (235) est supérieur au diamètre interne (210) à la deuxième position (220) et la deuxième position (220) se trouve entre la troisième position (235) et la première position (215).
